# EUROPÄISCHE PATENTSCHRIFT

(11) **EP 3 551 044 B1**
(45) Veröffentlichungstag und Bekanntmachung des Hinweises auf die Patenterteilung: **16.10.2024**
(21) Anmeldenummer: 17821776.6
(22) Anmeldetag: 04.12.2017
(51) Int. Cl.: A61B 5/00, A61B 5/0205, A61B 5/16, A61B 5/01

(54) **EINRICHTUNG ZUR UNTERSUCHUNG EINES MENSCHLICHEN PATIENTEN**
APPARATUS FOR EXAMINING A HUMAN PATIENT
DISPOSITIF SERVANT À L'EXAMEN D'UN PATIENT HUMAIN

(30) Priorität: 06.12.2016 AT 511092016
(43) Veröffentlichungstag der Anmeldung: 16.10.2019
(73) Patentinhaber: Schletterer Consult GmbH, 6235 Reith im Alpbachtal (AT)
(72) Erfinder: SCHLETTERER, Heinz, 6235 Reith im Alpbachtal (AT)
(74) Vertreter: Torggler & Hofmann Patentanwälte - Innsbruck
(86) Internationale Anmeldenummer: PCT/AT2017/060320
(87) Internationale Veröffentlichungsnummer: WO 2018/102840

(56) Entgegenhaltungen:
- WO-A1-2016/074036
- US-A- 5 441 047
- US-A- 5 640 953
- US-A1- 2002 123 704
- US-A1- 2003 216 665
- US-A1- 2004 178 312
- US-A1- 2004 254 501
- US-A9- 2007 058 035

## Beschreibung

Die Erfindung betrifft eine Einrichtung zur Untersuchung eines menschlichen Patienten mit zumindest zwei, vorzugsweise mehreren unterschiedlichen Messvorrichtungen, die Messdaten bezüglich des Zustandes des Patienten ausgeben.

Einrichtungen zur Untersuchung eines menschlichen Patienten sind bisher hauptsächlich so gehandhabt worden, dass für einzelne körperliche Untersuchungen verschiedene Messungen zeitlich hintereinander vorgenommen wurden und dann die Befunde zu einer Diagnose zusammengetragen worden sind. Die gleichzeitige Berücksichtigung des psychischen Zustandes des Patienten durch Zusammenschau der körperlichen und psychischen Befunde kommt immer mehr Bedeutung zu. Einschlägige Einrichtungen sind beispielsweise aus den Dokumenten US 2002/123704 A1, US 2004/254501 A1 und WO 2016/074036 A1 bekannt, bei denen jeweils körperliche und mentale Messdaten zueinander in Beziehung gesetzt werden. Ähnliche Einrichtungen sind außerdem aus den Dokumenten US 2004/178312 A1, US 2003/216665 A1, US 2007/058035 A9 und US 5640953 A bekannt, in denen weitere konstruktive Details solcher Einrichtungen offenbart werden.

Aufgabe der Erfindung ist es, eine Einrichtung zur Untersuchung eines menschlichen Patienten zu schaffen, mit dem es in kurzer Zeit möglich ist, ein gutes Gesamtbild vom Gesundheitszustand des Patienten zu erlangen.

Erfindungsgemäß wird dies durch die Merkmale des Anspruchs 1 erreicht.

Eine wesentliche Eigenschaft der Erfindung besteht darin, eine Wechselbeziehung zwischen den Messdaten neben den eigentlichen Messdaten selbst auszuwerten. Erst durch diese Korrelation der Messdaten, ist es häufig möglich, zu einer Beurteilung des gesundheitlichen Gesamtbildes zu kommen. Vor allem, wenn ein Teil der korrelierten Messdaten körperliche Messdaten sind, während der andere Teil mentale Messdaten sind. Damit können körperliche und psychische Zustände des Patienten korreliert werden und in Abhängigkeit von dieser Korrelation eine Befundung und/oder eine automatisierte Steuerung von Therapiegeräten erfolgen.

Damit ist die Diagnostik und Therapie in kurzen Zeiträumen wesentlich erleichtert.

Weitere Vorteile und Einzelheiten der Erfindung werden anhand der nachfolgenden Figurenbeschreibung näher erläutert.
Die Figur 1 zeigt ein Ausführungsbeispiel einer erfindungsgemäßen Einrichtung in einer schematischen Darstellung
Die Figur 2 zeigt verschiedene "biologische "Screening-Positionen" zum Erfassen von körperlichen Messdaten des auf einem Träger liegenden Patienten.
Die Figur 3 zeigt "psychologische, neurologisch und mentale Screening-Positionen" zum Erfassen von psychischen (mentalen) Messdaten des auf dem Träger sitzenden oder liegenden Patienten.
Die Figur 4 zeigt ein Ausführungsbeispiel einer erfindungsgemäßen Einrichtung in einem schematischen Block-Schalt-Diagramm.
Die Figur 5 zeigt in einer schematischen Übersicht die "biologischen Scans" zum Ermitteln von körperlichen Messdaten und "mentale Scans" zum Ermitteln von mentalen Messdaten, die den psychischen Zustand des Patienten wiedergeben.
Die Figuren 6 und 7 zeigen in gesonderten Darstellungen das biologische Screening zum Ermitteln von körperlichen Messdaten und das psychologische Screening zum Ermitteln von mentalen Messdaten, die den psychischen Zustand des Patienten wiedergeben.

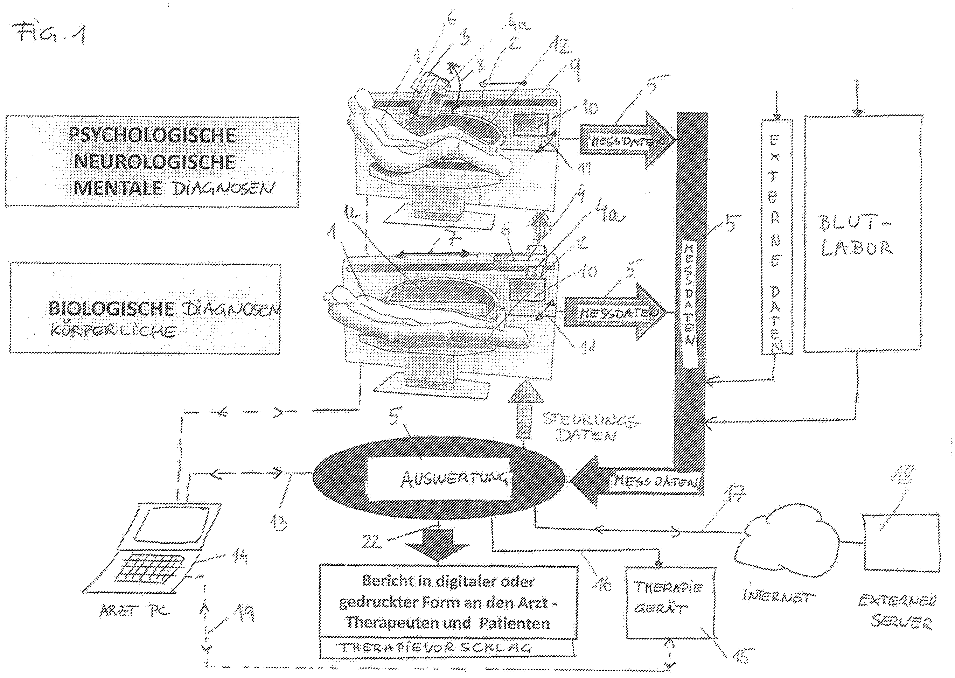

Die Figur 1 zeigt ein Ausführungsbeispiel einer erfindungsgemäßen Einrichtung zur Untersuchung eines menschlichen Patienten 1, der auf einem Träger 2 Platz nehmen kann.

Die beiden übereinander liegenden Darstellungen der verschiedenen Positionen des Trägers 2 betreffen ein- und dieselbe Einrichtung zu zwei verschiedenen Zeiten. In der oberen Position werden psychologische, neurologische, mentale Diagnosen erstellt, in der unteren Position biologische, körperliche Diagnosen.

Es ist eine als Bildschirm ausgebildete Anzeigevorrichtung 3 vorgesehen, die von dem auf dem Träger 2 befindlichen Patienten einsehbar ist (obere Stellung in Figur 1). Weiters ist eine Messvorrichtung 4 zum Erfassen von Messdaten des Patienten 1 sowie eine Auswerteinrichtung 5, die mit der Anzeigevorrichtung 3 und der Messvorrichtung 4 über schematisch dargestellte Leitungen 5 in Verbindung steht.

Beim vorliegenden Ausführungsbeispiel ist sowohl die Anzeigevorrichtung 3 als auch zumindest ein Sensor 4a der Messvorrichtung 4 gegenüber dem Träger 2 für den Patienten 1 beweglich gelagert.

Beim dargestellten Ausführungsbeispiel ist die Messvorrichtung ein Thermoscanner, wobei der Sensor 4a ein Infrarotsensor ist.

Mit einem solchen Thermoscanner lassen sich verschiedenste körperliche Diagnosen durchführen, insbesondere können Entzündungsherde, aber auch andere Veränderungen aufgezeigt werden.

Um den ganzen Körper zu erfassen, ist die Messvorrichtung in Form des Thermoscanners auf einem Halter 6 in Form eines Schlittens montiert und in Richtung der Pfeile 7 längsbeweglich linear geführt.

Der Halter 6 ist ein gemeinsamer Halter für den Thermoscanner 4 und die Anzeigevorrichtung 3. Um den Halter aus der Scanposition in der Mitte der Figur 1 in die obere Position in Figur 1 zu bringen, kann dieser in Richtung der Pfeile 8 verschwenkt werden, womit der Patient Einblick auf die Anzeigevorrichtung bzw. den Bildschirm erlangt. Konstruktiv einfach lässt sich diese in Figur 1 gezeigte Lösung dadurch realisieren, dass der Halter 6 einen verfahrbaren Basisteil und einen an diesem schwenkbar gelagerten Schwenkteil aufweist, der die Anzeigevorrichtung 3 und die Messvorrichtung 4 bzw. deren Sensor 4a trägt.

Der Anzeigevorrichtung kann auch noch mindestens ein (nicht dargestellter) Lautsprecher zugeordnet sein. Dieser kann entweder am feststehenden Teil der Einrichtung oder am verfahrbaren Schlitten angeordnet sein. Es ist auch möglich, dass der Lautsprecher in einem Kopfhörer untergebracht ist.

Insgesamt kann in der in Figur 1 oben gezeigten Position dem Patienten ein audiovisuelles Bild oder eine audiovisuelle Bilderfolge präsentiert werden und anschließend bzw. auch gleichzeitig Messdaten des Patienten erfasst werden. Es handelt sich dabei um reaktive Messdaten, die Aufschluss über den psychologischen Gesundheitszustand des Patienten geben, beispielsweise kann man den Patienten Stresssituationen und Konfliktsituationen vorspielen und seine Reaktion überprüfen. Man kann auch Farben vorspielen und seine Reaktion überprüfen.

Es ist auch möglich, psychologische digitale Fragebögen zu präsentieren, worauf der Patient dann über eine Eingabevorrichtung die Antworten eingeben kann.

Besonders bevorzugt ist eine Ausführungsform, bei der der Bildschirm 3 als Touch-Screen ausgebildet ist und somit gleichzeitig als Eingabevorrichtung dienen kann.

Während der psychologischen Untersuchung oder knapp danach, kann beispielsweise über eine zum Eyetracking geeignete Kamera und/oder ein Mikrophon zur Aufnahme von Lauten des Patienten weitere mentale Messdaten aufgezeichnet werden.

Diese Messdaten können bei hintereinanderliegenden Untersuchungen auch zwischengespeichert werden, um die weiter unten beschriebene Korrelation mit anderen Daten durchführen zu können.

Um einen vollautomatisierten Betrieb mit gleichbleibenden Ergebnissen zu erzielen, ist bevorzugt vorgesehen, dass die Anzeigevorrichtung 3 und der Sensor 4a der Messvorrichtung 4 motorisch bewegbar an einer Tragvorrichtung 9 gelagert sind, die in einem optisch ansprechenden Gehäuse untergebracht sein kann.

Die Tragvorrichtung 9 kann auch den Träger 2 für den Patienten tragen. Es ist aber auch möglich, dass dieser Träger 2 wie in Figur 1 dargestellt, direkt auf dem Boden aufsteht. Dann ist die Tragvorrichtung bzw. das Gehäuse 9 und der Träger 2 für den Patienten über den Boden des Raumes miteinander in fixer räumlicher Relation zueinander angeordnet.

Um den Patienten auch während biologischer körperlicher Diagnosen, bei denen der Schlitten als Thermoscanner arbeitet, einen Einblick auf seinen Gesundheitszustand und den Fortschritt der Untersuchung zu ermöglichen, eine weitere Anzeigevorrichtung 10 vorgesehen sein, die in Pfeilrichtung 11 aus dem Gehäuse herausschwenkbar ist bzw. in unbenutztem Zustand wieder bündig mit diesem einschwenkbar ist.

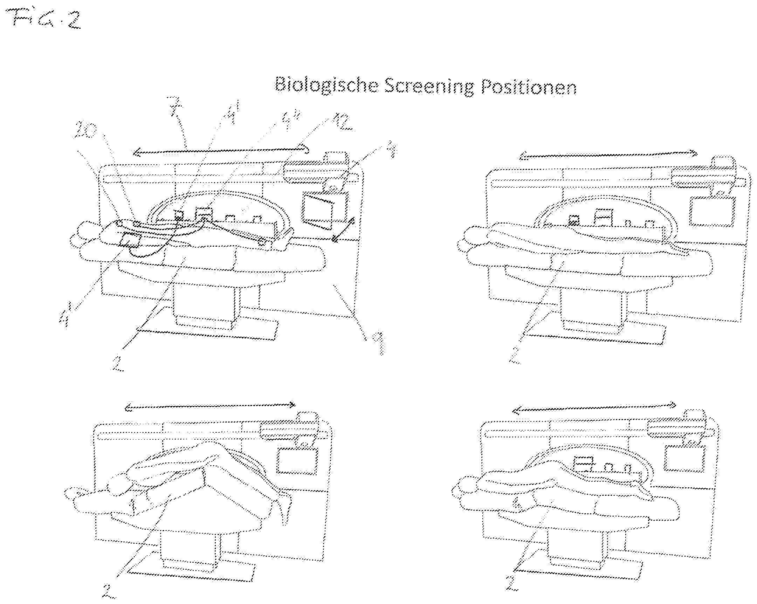

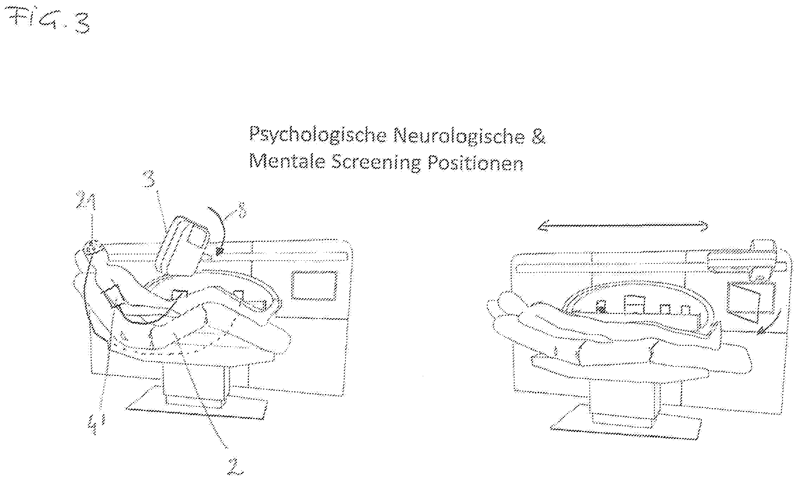

Beim dargestellten Ausführungsbeispiel gibt es dann noch in der Tragvorrichtung 9 bzw. im Gehäuse derselben eine Aufnahme 12, die von der Patientenseite her gut zugänglich ist. In dieser Aufnahme 12 können weitere Sensoren anderer medizinischer Messvorrichtungen untergebracht werden, wie das anhand der folgenden Figuren 2 und 3 noch näher beschrieben werden wird.

Beim dargestellten Ausführungsbeispiel ist der Träger 2 für den Patienten 1 als mehrteilige Sitz-Liege ausgebildet, deren Teile in ihrer Lage motorisch verstellbar sind.

Die Sitzliege ist bevorzugt dreiteilig ausgebildet mit einem Brustteil, einem Mittelteil und einem Fußteil, wie das die Figuren 1 bis 3 klar zeigen.

Die Figur 1 zeigt auch, dass die Sitzliege in eine Sitzstellung (obere Stellung in Figur 1) bringbar ist, wobei die Anzeigevorrichtung heruntergeschwenkt ist, sodass der Kopf des Patienten im Wesentlichen auf derselben Höhe liegt, die die Anzeigevorrichtung. Das entspricht im Wesentlichen der normalen Fernseh- bzw- Bildschirmbetrachtposition. Eine Alternative wäre jene, bei der die Sitzliege und die Anzeigevorrichtung in eine Stellung bringbar sind, in der der Patient im Wesentlichen geradeaus auf die Anzeigevorrichtung blickt. Das wäre beispielsweise dann möglich, wenn der Patient flach liegt und dennoch auf dem Bildschirm etwas sehen soll.

Die Einrichtung zur Untersuchung des menschlichen Patienten 1 weist zumindest zwei, vorzugsweise mehrere unterschiedliche Messvorrichtungen auf, die jeweils Messdaten bezüglich des Zustandes des Patienten abgeben. Es ist weiters eine elektronische Auswerteinrichtung 5 vorgesehen, die in Abhängigkeit von aus zumindest zwei unterschiedlichen Messvorrichtungen stammenden Messdaten und deren Wechselbeziehung (Korrelation) Ausgangssignale ermittelt, die dann, wie die Figur 1 zeigt, beispielsweise über eine Leitung 13 einem für einen Arzt vorgesehenen Rechner (Arzt-PC 14) zuführbar sind. Die Ausgangssignale können auch als Steuerdaten für eines oder mehrere Therapiegeräte 15 über eine Leitung 16 zugeführt werden.

Außerdem können die Ausgangssignale in einem Bericht in digitaler oder gedruckter Form an den Arzt oder Therapeuten bzw. Patienten gegebenenfalls zusammen mit einem Therapievorschlag weitergegeben werden.

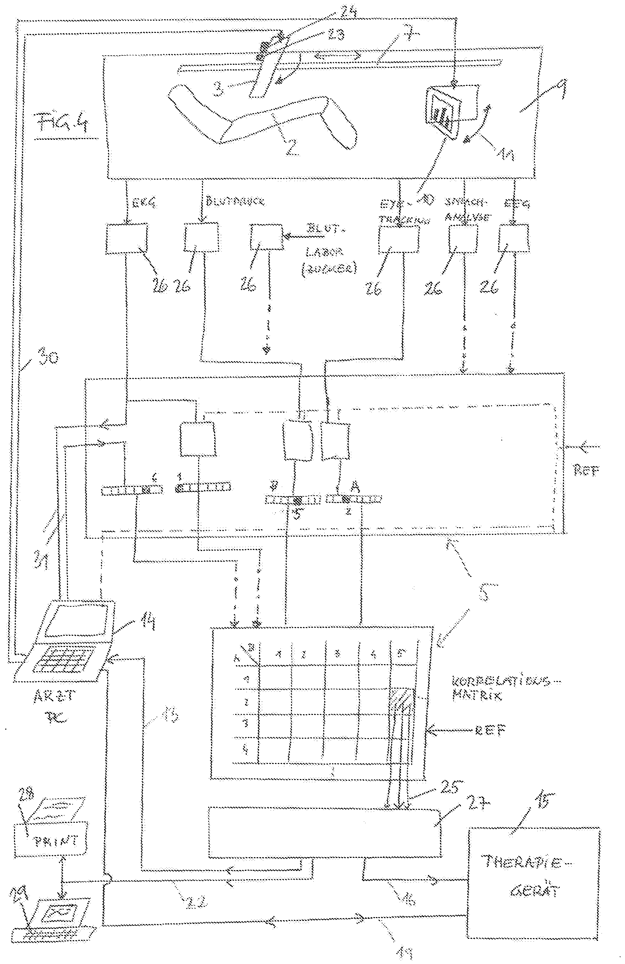

Weitere Details der Auswerteinrichtung werden weiter unten anhand der Figur 4 beschrieben.

Die Auswerteinrichtung kann jedenfalls auch teilweise dezentral arbeiten, wobei über eine schematisch dargestellte Leitung 17 und nicht näher dargestellte Kommunikationsmittel (beispielsweise über das Internet) auch ein an einem anderen Ort aufgestellter externer Server ansprechbar ist. In diesem externen Server kann einerseits Rechnerleistung erbracht werden, aber vor allem auch Referenzdaten abgespeichert werden, die es erlauben, die erfassten Messdaten des Patienten im Sinne einer Diagnose auszuwerten. Jedenfalls bleibt aber auch bei einer automatischen Therapie (Therapiegerät 15, Leitung 16) oder einem Therapievorschlag immer auch der Arzt beispielsweise über die Leitung 19 derjenige, der in den Therapievorschlag bzw. die automatische Therapie eingreifen kann.

Die Figur 2 zeigt einerseits verschiedene Position des als Sitzliege ausgebildeten Trägers für den Patienten, die je nach Untersuchungstyp motorisch eingestellt werden kann.

In Figur 2 links oben liegt der Patient auf dem Rücken. Es wird über ein Blutdruckmessgerät 4' mit Armmanschette und Messdatenaufbereitung der Blutdruck erfasst. Über ein EKG-Gerät 4" mit Elektroden 20 kann ein Elektrokardiogramm aufgezeichnet werden. Gleichzeitig oder danach kann ein Bild mit dem Thermoscanner 4 erzeugt werden, der in Richtung des Doppelpfeiles 7 linear bewegbar ist. Es ist aus der Figur 2 ersichtlich, dass die Tragvorrichtung 9 eine Aufnahme 12 (in optisch ansprechender elliptischer Form) aufweist, in der die diversen Messvorrichtungen bzw. deren Sensoren untergebracht sind.

Wie die einzelnen Abbildungen in Figur 2 zeigen, müssen nicht immer alle Messgeräte angeschlossen werden. Es können beispielsweise nur Scans mit dem Thermoscanner durchgeführt werden, um die körperlichen Daten aufzuzeichnen. Günstiger und bevorzugt ist allerdings vorgesehen, dass gleichzeitig oder knapp hintereinander mehrere Messungen vorgenommen werden. Dazu können insbesondere folgende Messvorrichtungen zum Einsatz kommen:
- eine Elektrokardiographie-Einrichtung (EKG),
- eine Einrichtung zur Feinstrommessung,
- eine Einrichtung zur Messung der Sauerstoffsättigung im Blut,
- eine Infrarot-Thermoscan-Einrichtung
- eine Einrichtung zum Blutdruckmessen,
- eine Einrichtung zur Messung der Herzratenvariabilität (HRV),
- eine Einrichtung zum Analysieren des Blutes,
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Einrichtung zur Untersuchung der Haut und/oder der Zähne mittels bildgebender Erfassung,
- eine Einrichtung zur Untersuchung und Analyse der Atemluft,
- eine Ultraschall-Messvorrichtung,
- eine Elektroenzephalographie-Einrichtung (EEG),
- eine Elektromyographie-Einrichtung (EMG),
- eine Elektroneurographie-Einrichtung (ENG),
- eine vom Patienten bedienbare Eingabevorrichtung,
- eine Einrichtung zur Video-Erfassung des Ganges des Patienten.

Diese Einrichtungen sind im technischen Aufbau und Ihrer Anwendung im Einzelnen dem Fachmann bereits bekannt und brauchen hier nicht näher beschrieben zu werden. Neuartig an der Erfindung ist allerdings, dass diese Geräte in einer größeren Zahl und knapper hintereinander eingesetzt werden können, wenn ein Gesamtbild des Gesundheitszustandes des Patienten erwünscht ist.

Es ist ein Merkmal der Erfindung, dass neben den körperlichen Daten in den biologischen Screeningpositionen gemäß Figur 2 auch mentale Messdaten erfasst werden, die den psychischen Zustand des Patienten wiedergeben. Das ist schematisch in Figur 3 dargestellt. In Figur 3 links sieht man den Träger 2 in einer Sitzposition. Die Anzeigevorrichtung 3 in Form eines Touchscreens ist in Pfeilrichtung 8 heruntergeschwenkt und kann vom Patienten eingesehen werden. Während dem Patienten hier audiovisuelle Daten vorgespielt werden, wird über eine schematisch dargestellte EEG-Einrichtung 21 sowie über das Blutdruckmessgerät 4' Gehirnströme und Blutdruck erfasst. Damit kann man reaktive Daten erzielen, die über den psychischen Zustand des Patienten Aufschluss geben. Auch in einer Liegeposition, wie sie in Figur 3 rechts dargestellt ist, kann beispielsweise durch Beschallung oder durch Farbwechsel im Raum der Patient beeinflusst werden und festgestellt werden, welche Reaktionen dann erfasste Messdaten aufweisen. Der Übersichtlichkeit halber sind in den Figuren 2 und 3 nicht alle möglichen Anschlüsse von Messvorrichtungen gezeigt. Prinzipiell können alle oben in der Liste angegebenen Geräte teilweise, hintereinander und großteils auch gleichzeitig eingesetzt werden.

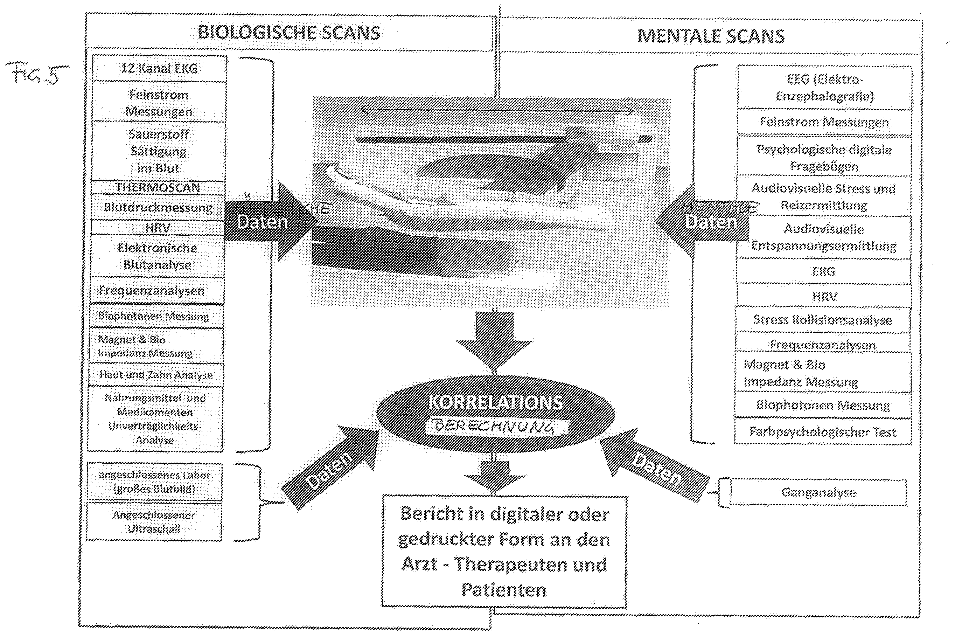

Erfindungsgemäß ist jedenfalls vorgesehen, dass der elektronischen Auswerteinrichtung 5 einerseits mentale Messdaten, die den psychischen Zustand des Patienten wiedergeben und andererseits körperliche Messdaten, die den körperlichen Zustand des Patienten wiedergeben, zugeführt werden, wobei die elektronische Auswerteinrichtung 5 aus den mentalen Messdaten und den körperlichen Messdaten und deren Wechselbeziehung (Korrelation) Ausgangssignale ermittelt, die dann über Leitungen 13, 16, 17, 22 weitere Funktionen erfüllen können, wie dies bereits anhand der Figur 1 kurz beschrieben worden ist und anhand der Figuren 4 und 5 noch näher beschrieben werden wird.

Bei dem in Figur 4 dargestellten Ausführungsbeispiel die Auswertung der Messdaten und die korrelative Ermittlung von Ausgangssignalen anhand eines vereinfachten Beispiels näher beschrieben.

Ganz oben in Figur 4 sieht man schematisch den Aufbau der mechanischen Komponenten der erfindungsgemäßen Einrichtung mit dem als Sitzliege ausgebildeten Träger 2. Der Patient ist aus Ubersichtlichkeitsgründen nicht dargestellt. In einem ersten Schritt kann die Sitzliege 2 in eine Liegeposition gebracht werden und über hier nicht näher dargestellte, aber allgemein bekannte Messvorrichtungen bzw. Sensoren ein EKG erstellt, Blutdruck gemessen und auch in einem Blutlabor bestimmte Parameter, beispielsweise der Blutzucker festgestellt werden. Dabei handelt es sich im Wesentlichen um körperliche Messdaten, die dann der elektronischen Auswerteinrichtung 5 zugeführt werden.

In der in Figur 4 dargestellten Sitzstellung der Sitzliege 2 können dem Patienten über den Bildschirm 2 und einem nicht dargestellten Lautsprecher audiovisuelle Bilder vorgeführt werden. Seine Reaktion kann dann beispielsweise über eine Kamera 22 (Eyetracking) erfasst werden. Auch möglich ist eine Sprachanalyse, in dem der Patient angehalten wird, beispielsweise einen vorgegeben Text oder eine Antwort auf eine bildlich dargestellte Frage zu beantworten. Das Mikrophon 24 nimmt die Sprache auf und kann eine Sprachanalyse vornehmen. Auch ein Elektroenzephalogramm (EEG), wie es beispielsweise in der Figur 3 links dargestellt ist, kann erstellt werden. Schließlich ist es auch möglich, dem Patienten über den Bildschirm Fragen zu stellen, die er dann durch Eingabe in den Bildschirm beantwortet. All diese letztgenannten Messdaten sind mentale Messdaten, die den psychischen Zustand des Patienten wiedergeben.

In Figur 4 ist anhand eines vereinfachten Beispiels dargestellt, wie aus einer Korrelation eines Typs von körperlichen Messdaten (hier der Blutdruck B) mit einem Satz von mentalen Messdaten (hier Eyetracking A) zu definierten Ausgangssignalen auf den Ausgangsleitungen 25 der elektronischen Auswerteinrichtung 5 führt.

Ebenfalls vorhandene weitere Messdaten bzw. Auswertungen sind hier in strichpunktierten Linien dargestellt aber nicht weiter ausgeführt, um die Darstellung nicht zu kompliziert zu halten und das Wesen der im folgenden beschriebenen Korrelationsmatrix herauszuarbeiten.

Zunächst werden alle Messdaten in zugeordneten Speichereinrichtungen 26 zwischengespeichert, weil es im Allgemeinen nicht möglich ist, die körperlichen Daten auf den drei linken Leitungen in Figur 4 oben gleichzeitig mit den mentalen Messdaten auf den drei rechten Leitungen in Figur 4 gleichzeitig zu erfassen und damit zur Verfügung zu haben.

Über die Zwischenspeicher 26 können beliebige auch seriell hintereinander aufgenommene Messdaten gleichzeitig zur Verfügung stehen.

Am Beispiel des Satzes von körperlichen Messdaten Blutdruck B und des Satzes der mentalen Messdaten Eyetracking A, wird nun in der elektronischen Auswerteinrichtung zunächst eine zahlenmäßige Bewertung vorgenommen, wobei beim dargestellten Ausführungsbeispiel der Blutdruck mit der Zahl 5 und die Eyetracking-Messung mit der Zahl 2 bewertet wurde.

Diese zwei Zahlen kommen nun in eine Korrelationsmatrix, wobei jedes Feld der Korrelationsmatrix einem Wertepaar W, A einen eindeutigen Wert des Ausgangssignals zuordnet wobei dies, wie beim vorliegenden Beispiel dargestellt, auch mehrere Werte sein können, beispielsweise drei Ausgangswerte auf den Ausgangsleitungen 25. Beispielsweise kann es sich um drei Werte handeln, die ein Therapiegerät 15 über die Sammelleitung 16 ansteuern. Es kann aber auch in der Ausgabeeinheit 27 zu einer Aufbereitung der Werte für eine Ausgabe auf einen Drucker 28 oder einen Bildschirm 29 kommen. Auch eine Aufbereitung für den Arzt WC 14 und Übermittlung der Daten der Überleitung 13 ist möglich.

Der Arzt kann dann entscheiden, ob er die Daten insgesamt oder teilweise mit dem Patienten teilen will und gegebenenfalls diese auf dem Zusatzbildschirm 10 darstellen. Der Arzt kann überhaupt über die Leitung 30 den gesamten Messdatenerfassungsprozess, insbesondere den Scanprozess steuern. Der Arzt-PC 14 kann auch über drahtgebundene oder drahtlose Verbindungen mit anderen Komponenten, insbesondere der elektronischen Auswerteinrichtung 5 kommunizieren. Er kann auch über das Internet mit dezentralen externen Servern verbunden sein. Dasselbe gilt für die elektronische Auswerteinrichtung, die selbst direkt über das Internet mit Referenzdaten REF aus einer externen Quelle versorgt werden kann. Das ist insbesondere auch über das Internet möglich.

Diese Referenzdaten erlauben es unter anderen, die geschilderte zahlenmäßige Bewertung der Messsignale, hier Blutdruck und Eyetracking B, A vorzunehmen und einzuordnen.

Weiters ist es auch möglich, dass die Werte in der Korrelationsmatrix über einen externen Referenzeingang REF bestimmt werden. Es ist aber auch möglich, dass in der Korrelationsmatrix eine Funktion hinterlegt ist, die aus den Zahlen ausgedrückten Messdaten und einem bekannten funktionellen Zusammenhang das entsprechende Matrix-Element oder den Satz von Matrix-Elementen ermittelt und über die Ausgabeleitungen 25 ausgibt.

Beim dargestellten Ausführungsbeispiel ist die Matrix lediglich zweidimensional, weil es sich nur um zwei dargestellte herausgegriffene Messdaten gehandelt hat.

Die Matrix wird bei mehreren Messdaten aber selbstverständlich dreidimensional ausfallen (was dann aber grafisch nicht mehr dargestellt werden kann). In jedem Fall können hier aber gemäß einem bevorzugten Merkmal der Erfindung körperliche Messedaten bzw. deren zugeordnete Zahlenwerte und mentale Messdaten bzw. deren zugeordnete Zahlenwerte miteinander korreliert werden und zu Ausgangssignalen verarbeitet werden.

Anhand der Verarbeitung der Messsignale des EKGs sei noch dargestellt, dass eine Messung (hier EKG) unter anderem mehrere Teilergebnisse bringt, die dann gesondert als Messdatenwerte zahlenmäßig zur Verfügung stehen.

Beispielsweise lässt sich die Lage bestimmter Abschnitte des EKGs leichter automatisch ermitteln. Das ist im vorliegenden Beispiel in Figur 4 Mitte links dargestellt, wo die zahlenmäßige Bewertung zum Wert 1 geführt hat.

Andere Eigenschaften des EKGs erlauben manchmal keine automatische Bewertung.

Hier wird der Arzt über die "Beurteilungsleitungen 31" eingeschaltet. Er sieht dann am PC 14 das EKG und kann es zahlenmäßig beurteilen, beispielsweise mit der Zahl 6, wie es im vorliegenden Beispiel dargestellt ist. Anschließend kann wieder eine vollständig automatisierte Korrelation vorgenommen werden.

Erfindungsgemäß ist vorgesehen, dass die mentalen Messdaten einerseits und die körperlichen Messdaten andererseits zumindest teilweise aus unterschiedlichen Messvorrichtungen stammen, die für das jeweilige Aufgabengebiet besonders adaptiert sind. Es ist aber durchaus möglich, dass ein- und dieselbe Messvorrichtung für beide Zwecke, also zur Ermittlung von körperlichen Messdaten und zur Ermittlung von mentalen Messdaten verwendet wird. Erfindungsgemäß ist eine Einrichtung zur nicht invasiven und berührungslosen Beeinflussung des Patienten vorgesehen und zumindest eine Messvorrichtung dazu ausgelegt, die Reaktion des Patienten zu erfassen und davon abhängige reaktive Messdaten auszugeben, die dann als mentale Messdaten verwendet werden.

Beispielsweise kann der Patient über Bildfolgen oder Videos beeinflusst werden und die Reaktion am EKG angesehen werden, um mentale Messdaten zu erhalten. Im Ruhezustand kann dasselbe EKG körperliche Messdaten erfassen.

Zur Erfassung von körperlichen Messdaten eignen sich besonders folgende Messvorrichtungen:
- eine Elektrokardiographie-Einrichtung (EKG),
- eine Einrichtung zur Feinstrommessung,
- eine Einrichtung zur Messung der Sauerstoffsättigung im Blut,
- eine Infrarot-Thermoscan-Einrichtung
- eine Einrichtung zum Blutdruckmessen,
- eine Einrichtung zur Messung der Herzratenvariabilität (HRV),
- eine Einrichtung zum Analysieren des Blutes,
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Einrichtung zur Untersuchung der Haut und/oder der Zähne mittels bildgebender Erfassung,
- eine Einrichtung zur Untersuchung und Analyse der Atemluft,
- eine Ultraschall-Messvorrichtung,
- eine Elektromyographie-Einrichtung (EMG).

Erfindungsgemäß stammen die mentalen Messdaten aus zumindest einer der folgenden Messvorrichtungen:
- eine Elektrokardiographie-Einrichtung (EKG),
- eine Einrichtung zur Feinstrommessung,
- eine Einrichtung zur Messung der Herzratenvariabilität (HRV),
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Elektroenzephalographie-Einrichtung (EEG),
- eine Elektroneurographie-Einrichtung (ENG),
- eine vom Patienten bedienbare Eingabevorrichtung,
- eine Einrichtung zur Video-Erfassung des Ganges des Patienten.

Erfindungsgemäß werden als mentale Messdaten reaktive Messdaten verwendet.

Ein wesentlicher Vorteil der erfindungsgemäßen Einrichtung besteht darin, dass sie in der Lage ist, mentale und körperliche Messdaten miteinander zu verknüpfen und in Abhängigkeit von dieser Korrelation Ausgangssignale zur Verfügung stellt. Damit lässt sich ein umfassendes Gesamtbild des Gesundheitszustandes des Patienten erzielen und dieses auch an den Arzt, Therapeuten und Patienten kommunizieren. Außerdem ist es möglich, diese Daten als Steuersignale für angeschlossene Therapievorrichtungen zu verwenden.

In Figur 5 sind nochmals im Überblick die wesentlichen biologischen Scans zur Ermittlung von körperlichen Messdaten links und die mentalen Scans zur Ermittlung von mentalen Messdaten rechts im Überblick dargestellt.

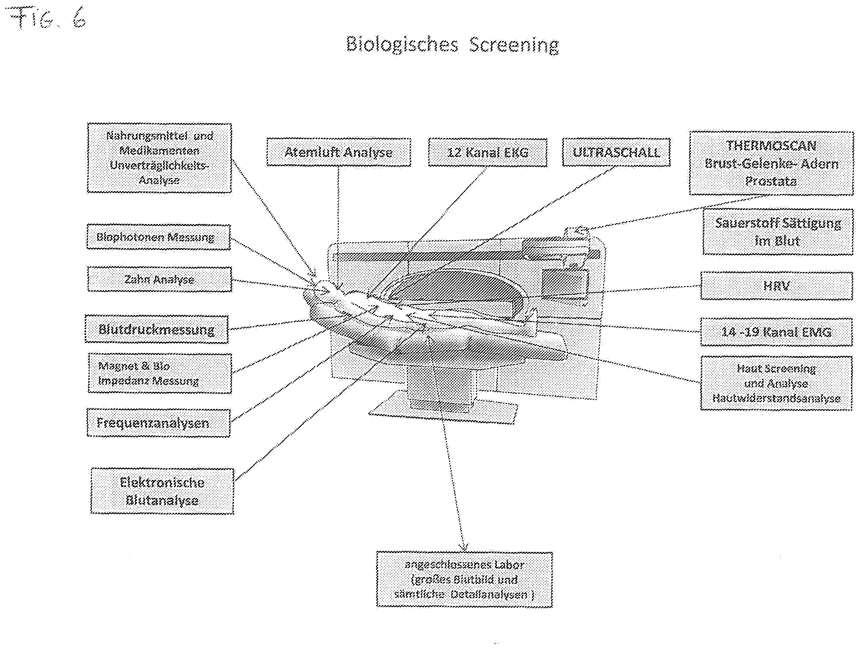

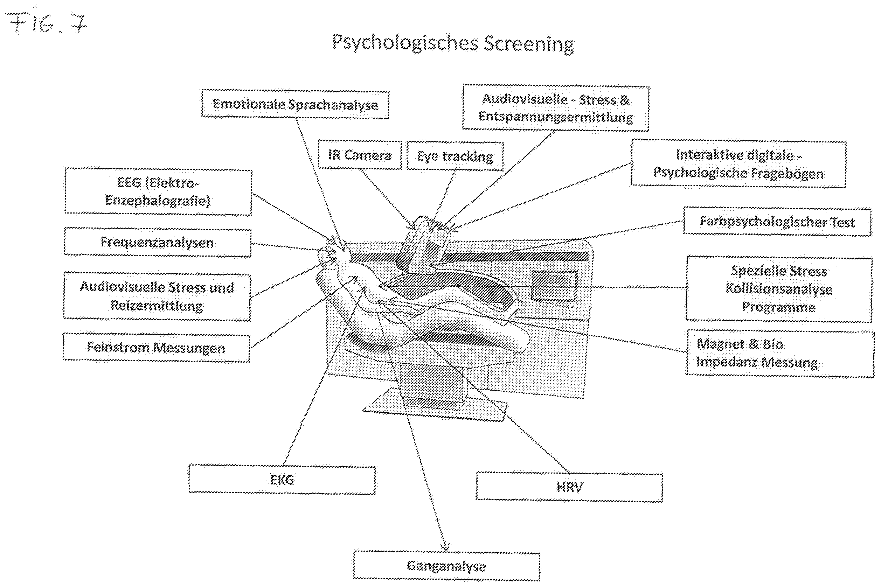

Die Figuren 6 und 7 zeigen in gesonderten Darstellungen das biologische Screening zum Ermitteln von körperlichen Messdaten und das psychologische Screening zum Ermitteln von mentalen Messdaten, die den psychischen Zustand des Patienten wiedergeben.

Insgesamt ist eine technische Verarbeitung aller gleichzeitig und in kurzer Zeit ermittelten biologischen und psychologischen Gesundheitsparametern samt Korrelationsüberprüfung möglich. Ausgaben erfolgen an elektrotechnische und digitale Geräte, Anweisungen an die verschieden angeschlossenen therapeutischen Equipments gegeben werden.

Die "Full Body Screening"-Anlage ist in der Lage, die in ganz kurzer Zeit ermittelten und analysieren Patienten Daten aus seinem biologischen und mentalen bzw. psychologischen Bereich nach der erfolgten Korrelationsberechnung, die auch über einen zentralen Server erfolgen kann, in eine technische Maschinensprache umzusetzen und an die verschiedensten therapeutischen Equipments automatisiert zu übermitteln.

Dabei werden sowohl audiovisuelle- als auch elektrotechnische Signale an die verschiedenen Equipments transferiert.

Mit "Wenn Dann" Befehlen werden die angeschlossenen Equipments angesteuert, wobei der Arzt oder Therapeut jederzeit die Möglichkeit hat in den Therapieverlauf einzugreifen, sollte er das für erforderlich halten.

Die verschiedenen Equipments wiederum senden die Ergebnisse nach durchgeführten Therapien zurück zum zentralen Server bzw. zur Auswerteinrichtung Diese Ergebnisprüfung wird durch in den Equipments eingebauten Sensoren wie beispielweise HRV - EEG - EKG - Magnetimpedanzmessungen - Feinstrom Messungen - Bewegungssensoren - Blutdruck und Blutsauerstoff Messungen - Frequenz Messungen usw. ermittelt.

Damit erhält der Arzt zusätzlich die Informationen, wie die Therapien angeschlagen haben.

Die Daten können auch "just in time" an ein zwischengeschaltetes telemedizinisches Zentrum gesendet werden und dort von Spezialisten und medizinischem Fachpersonal gecheckt werden.

In der Patienten-Datenbank im Zentralserver wird bevorzugt nach der Behandlung ein weiterer Korrelationslauf durchgeführt um die Ergebnisse der Therapien mit den Ausgangsdaten abgleichen zu können und gegebenenfalls die Therapien adaptieren zu können.

Die Screening-Anlage ermittelt den Gesundheitsstatus:
- in einer bisher noch nie dagewesenen kurzen Zeit
- und in einer hohen Dichte an Daten und Informationen sowohl aus dem biologischen als
auch aus dem psychologischen bzw. dem mentalen Bereich inklusive dem Verhaltens- relevanten Bereich. Und das alles "non-invasiv" und bevorzugt ohne Strahlenbelastung.

Die verschiedenen Screening Ergebnisse werden in der Folge noch im Detail beschrieben.

Dadurch können die Untersuchungszeiten von mehreren Stunden und sehr oft von Tagen, insbesondere wenn verschiedenen Spezialisten bzw. Fachärzte aufgesucht werden müssen, eingespart werden. Der Screening Vorgang mit erfindungsgemäßen Anlage dauert in den meisten Fällen nur wenige Minuten. Bei schwerwiegenden Problem bis maximal 1 Stunde. Wertvolle Lebenszeit - verlorene Arbeits- oder Freizeit kann dadurch gewonnen werden.

Die Screening-Anlage ermittelt den Gesundheitsstatus der Menschen in einer noch die dagewesenen holistischen Art und Weise.

Dadurch werden bestehende oder erst entstehende Krankheiten aufgezeigt die mit einer normalen Untersuchung kaum und schon gar nicht mit diesem geringen Zeiteinsatz_entdeckt werden würden.

Die Screening-Anlage ist in der Lage nicht nur die Symptome sondern auch viele Ursachen und Fehlverhalten die zu einer Krankheit führen, aufzuspüren und gezielten Therapien zuzuführen.

Die meisten derzeitigen Analysen konzentrieren sich auf die Diagnose von erkennbaren Symptomen. Die erfindungsgemäße Anlage konzentriert sich neben einer hohen Diagnose-Genauigkeit vor allem auch auf die Ursachenermittlung, wobei einige Therapien von der Anlage direkt durchgeführt werden können.

Ein weiterer Vorteil der erfindungsgemäßen Anlage ist der Umstand, dass diese Anlage alle ermittelten Paramater mit zahlreichen Vergleichsdaten abgleichen kann und über einen speziellen Algorithmus, insbesondere Matrix-Berechnung, herausfindet welchen zukünftigen Verlauf der ermittelte Vitalitäts- bzw. Gesundheits- oder Krankheitsstatus nehmen wird. Dabei kann simuliert werden, wie sich der Zustand des Menschen verändert sofern er gleich weiterlebt bzw. was passiert wenn er seine Lebensweise ändert.

Darüber hinaus kann simuliert und dargestellt werden, welchen Einfluss verschiedene Medikamente und Naturheil- und Nahrungsmittel auf den untersuchten Menschen haben werden.

Damit kann die Lebensqualität verbessert und das Leben verlängert werden. Vor Allem können eventuell entstehende Krankheiten vermieden und bestehenden Krankheiten eliminiert werden.

Die Screening-Anlage kann auch Nahrungsmittel Unverträglichkeiten in einer in dieser Kombination noch nie dagewesenen Präzision erkennen. Dabei wird über ein Ganzkörper-Scanning über Photonen aus Gewebe und Magnetfeld und Energiedichtemessungen die generelle Unverträglichkeit geprüft. Und parallel dazu wird über Bluttests und einem Atemluft Test die Unverträglichkeit geprüft. Ein spezielle HRV (Herzratenvariabilität) Analyse vertieft und bestätigt das Testergebnis. Damit ergibt sich eine Analyse- und Diagnose-Qualität, die sonst nur mit sehr vielen verschiedenen Untersuchungen meist bei verschiedenen Spezialisten möglich ist.

Damit können die Essgewohnheiten der Menschen so abgestimmt werden, dass diese die optimale Ernährung genießen können ohne mit unverträglichen Lebensmitteln den Körper bzw. die Gesundheit zu belasten.

Die Anlage ist weiters in der Lage Unverträglichkeiten gegen Medikamente festzustellen und über die vielen in der Anlage integrierten Vergleichsparameter verträgliche Alternativen vorzuschlagen.

Diese Alternativen können zusätzlich durch weitere Screenings auf Ihre Verträglichkeit verifiziert werden.

Der Mensch bekommt genau jene Medikation, die für Ihn ideal ist. Unverträgliche Medikamente werden erkannt und können durch verträgliche Medikamente und Behandlungsmethoden ersetzt werden.

Insgesamt ist die Anlage in der Lage nicht nur exzellente Analysen und Diagnosen zu erstellen, sondern auch und das ist ebenfalls weltweit einzigartig, diese Analysen und Diagnosen mit verschiedenen Methoden direkt am Gerät zu therapieren oder Therapien automatisiert vorzuschlagen.

Der Nutzer der Anlage spart sich wiederum Zeit und verschiedene Wege zu diversen Spezialisten und kann sofort umfassend und nach modernsten Erkenntnissen therapiert werden.

Die über die Anlage ermittelten Daten und Fakten können in Echtzeit an ein telemedizinisches Zentrum weitergeleitet werden.

Das Analyse- und Diagnose-Programm der Screening Anlage stellt nicht nur all Gesundheits- bzw. Krankheitsfakten dar Fakten dar, sondern schlägt auch gleichzeitig die bestmöglichen Behandlungsmethoden vor.

Im Telemedizin Zentrum oder vor Ort neben der Anlage kontrolliert ein Ärzte Team diese Daten und Fakten und implementiert eine finale Empfehlung in den Statusbericht der IQCUREO Health Screening Anlage.

Der überprüfte Bericht geht nun an den behandelnden Arzt bzw. an das medizinische Fachpersonal vor Ort, die ja mit dem Patienten bzw. dem Gast in direkter Verbindung stehen.

Mit diesem kontrollierten und bei Bedarf durch das Telemedizinzentrum adaptierten Bericht bekommt das medizinische Fachpersonal eine perfekte Information um den Nutzer vor Ort zu betreuen.

Durch die hohe Qualität der ermittelten Daten und Fakten sowie der durch die Anlage erstellten Diagnosen und Behandlungsempfehlungen sowie durch die übergeordnete Kontrolle durch das Telemedizin Zentrum oder einen Arzt vor Ort, erhält das medizinische Fachpersonal die bestmöglichen Unterlagen um den Patienten/Gast zu behandeln und zu betreuen.

Gerade für noch junge und etwas unerfahrenere Ärzte ist diese Information extrem wichtig, um Fehldiagnosen und falsche Behandlungen möglichst zu verhindern.

Aber auch ein noch so erfahrender Arzt ist erstaunt und extrem positiv überrascht, welch wertvolle Informationen er über das erfindungsgemäße System erhalten kann. Auch der beste Arzt kann niemals diese ganzen Informationen in derart kurzer Zeit ermitteln.

Die Analysen, die Diagnosen und die Behandlungsempfehlungen, die über die Anlage an die Patienten übergeben werden, sind völlig neuartig, insbesondere die Tatsache. dass all diese Ergebnisse der Analysen, der Diagnosen, der Risiko-Parameter und auch die empfohlenen Behandlungen in einer für jedem Laien verständlicher Sprache übermittelt werden können.

Dabei wird diese Informationen mit anschaulichen bzw. erklärenden Grafiken und Videos unterstützt und vervollständigt.

Die gesamte Information als auch sämtliche medizinischen Fachausdrücke kann das System automatisch in die jeweilige Landessprache des Patienten/Gastes übersetzen. Der Arzt bzw. das medizinische Fachpersonal wiederum bekommt alle Daten in einem bekannten medizinischen Format zur Verfügung gestellt, wobei der Umfang der Information auf Grund Informationsdichte und der Daten Qualität einzigartig ist.

Die meisten Analysen, die der Mensch heute bekommt, sind gespickt mit Fachausdrücken die nur ein Arzt interpretieren kann. Damit ist eine enorme Verunsicherung vorprogrammiert.

Der Nutzer der Anlage bekommt sämtlich Informationen in einer verständliche zum Teil auch grafischer und bildhaften animierten Weise in seiner Landessprache erklärt und kann diese Information bestens verstehen und verwerten.

Der Mediziner bekommt wesentlich mehr Informationen über den Patienten/Gast und das in einer ganz kurzen Zeit und kann damit seine Behandlung wesentlich zielgerichteter ausrichten.

Durch die Zeitersparnis ergibt sich für den Mediziner darüber hinaus auch noch ein ganz wesentlich wirtschaftlicher Vorteil

Folgende Screenings können im Wesentlichen synchron während der Untersuchung in ganz kurzer Zeit durchgeführt und umfassende Daten ermitteln die dann unmittelbar darauf in eine Behandlungs- Empfehlung an das medizinische Personal übermittelt werden.

Allgemeine Gesundheits- bzw. Vitalitäts- Status Ermittlung
- Erhebung des Vitalitätsstatus im Vergleich zu Referenzgruppen.
- Unter Einsatz von Organ-Frequenz-Analysen
- Unter Einsatz von (HRV) Herzraten-Variabilitäts-Checks.
- Unter Einsatz von Photonen-Analysen aus menschlichem Gewebe.
- Unter Einsatz von Energie Dicht Messungen der einzelnen Organe.
- Ermittlung von Entzündungen im Körper
- Ermittlung von Onkologischen Problemen.
- Blutstatus Ermittlung
- Zahnstatus Ermittlung
- Atemluft-Status
- Hautstatus
- Entzündungsstatus
- Augenstatus
- Check sämtlicher Organe in Bezug auf eventueller Krankheiten und Risikofaktoren.
- Soll Ist Vergleiche in Bezug auf Mineral Mankos
- Ermittlung von Medikamenten Unverträglichkeiten
- Ermittlung von Lebensmittel Unverträglichkeiten
- EKG
- Sauerstoff Sättigung des Blutes.
- Erhebung des mentalen bzw. psychologischen Gesundheits- Status

Unmittelbar nach Erhebung des allgemeinen Gesundheits- und Vitalitäts- Status werden, sofern bei einzelnen Organen Irritationen erkannt wurden, die betroffenen Organe speziell gescannt und bei Bedarf ganz spezielle Therapie-Empfehlungen abgegeben.

Das gilt auch für den mentalen bzw. psychologischen Gesundheitsstatus sowie der Haut:
- Erhebung des Haut Durchblutungs- Status über hochsensible Thermografie Ermittlung von Entzündungen
- PH Wert Ermittlung der Haut in verschiedenen Hautzonen.
- Ermittlung von Hautirritationen
- Hochauflösende Aufnahme von Muttermalen und Hautirritationen die auf einen eventuellen Hautkrebs hinweisen und Abgleich mit tausenden Referenzbildern
- Ermittlung von Haut Allergien.

Zähne:
- Erhebung des Zahnstatus über Biophotonen und Energiedichte Messung
- Aufnahme des optischen Zahnstatus
- Kontrolle über Entzündungsmarker

Bezüglich Brustuntersuchungen ist beispielsweise folgendes möglich:
- Erhebung der Brustgesundheit über Thermografisches Screening mit Bildgebender Darstellung ohne jegliche Strahlenbelastung.
- Sichtbarmachung von Entzündungen und Brustirritationen auch in einem sehr frühen Stadium
- Doppel Check über Krebsmarker aus der Blutuntersuchung.
- Check des gesamten Bereichs über Biophotonen - Energie Dichte Messungen - HRV Messungen

Bezüglich Gelenken:
- Screening der Gelenke um Irritationen - Abnützungen - und Entzündungen feststellen zu können.

Dabei werden HRV Messungen, Photonen-Messungen, Energie-Dichte-Messungen und Thermosensoren mit Bildgebender Darstellung eingesetzt.

Bezüglich Männergesundheit:
- Der gesamte Männergesundheitsbereich wird analysiert. Dabei wird die Prostata - der Blasenbereich die Potenz und alles was mit der Männergesundheit zusammenhängt gescreent und diagnostiziert - sowie Behandlungen vorgeschlagen Männergesundheit:
   Bezüglich Frauengesundheit:
- Der gesamte Frauengesundheitsbereich wird analysiert. Dabei wird die Gebärmutter - der Blasenbereich (Inkontinenz) und alles was mit der Frauengesundheit zusammenhängt gescreent und diagnostiziert - sowie Behandlungen vorgeschlagen

Bezüglich Organen im Allgemeinen:
- Sofern bei den einzelnen Organen bei der Vitalstatus Erhebung - Auffälligkeiten entdeckt wurden wird jedes betroffene Organ detailliert gescreent und diagnostiziert und eine Behandlungsempfehlung ausgedruckt.

Dies ist auch der Grund warum die normalerweise sehr kurze Diagnosezeit sich auf bis zu eine Stunde verlängern kann. Wobei selbst eine Stunde nur ein Bruchteil des Zeitbedarfs ausmacht den man benötigt, um alle Organe bei den verschiedenen Fachärzten untersuchen zu lassen.

Zur mentalen und psychosozialen Gesundheit kann beispielsweise folgendes ermittelt werden:
- Mit speziellen Screening Verfahren die über bewegte Bilder (Kurzfilme) über Sprachausgaben über Texteingaben und parallel über Photonen und Energie-Dichte-Messungen über Reaktionsanalysen - (Schweiß und Körpertemperatur Messungen) sowie über HRV Kontrollmessungen erfolgen.
- Auch werden die möglichen Erregungs- und Entspannungszustände gemessen.
- Es werden die höchsten Stressfaktoren und die besten Entspannungsmethoden ermittelt und in einem automatisiert erstellten Therapiepaket dem Patienten/ Gast und dem medizinischen Fachpersonal übermittelt. Darüber hinaus wird ein personalisiertes Farbprofil erstellt und eine individuelle Farbtherapie automatisiert ausgearbeitet, die wiederum mit einer speziellen Aromatherapie Empfehlung verknüpft ist.
- Vor Allem werden die wesentlich Beziehungskollisions-Faktoren ermittelt die zu einer Stressbelastung führen.

## Patentansprüche

1. Einrichtung zur Untersuchung eines menschlichen Patienten (1), mit zumindest zwei, vorzugsweise mehreren unterschiedlichen Messvorrichtungen, die Messdaten bezüglich des Zustandes des Patienten ausgeben, wobei eine elektronische Auswerteinrichtung vorgesehen ist, die so ausgelegt ist, dass sie in Abhängigkeit von aus zumindest zwei unterschiedlichen Messvorrichtungen stammenden Messdaten und deren Wechselbeziehung Ausgangssignale ermittelt, wobei der elektronischen Auswerteinrichtung einerseits mentale Messdaten, die den psychischen Zustand des Patienten wiedergeben, und andererseits körperliche Messdaten, die den körperlichen Zustand des Patienten wiedergeben, zugeführt werden, wobei die elektronische Auswerteinrichtung (5) so ausgelegt ist, dass sie aus den mentalen Messdaten und den körperlichen Messdaten und deren Wechselbeziehung Ausgangssignale ermittelt,
wobei die mentalen Messdaten einerseits und die körperlichen Messdaten andererseits zumindest teilweise aus unterschiedlichen Messvorrichtungen stammen,
wobei eine Einrichtung zur nicht invasiven und berührungslosen Beeinflussung des Patienten vorgesehen ist und zumindest eine Messvorrichtung so ausgerichtet ist, dass sie die Reaktion des Patienten erfasst und davon abhängige reaktive Messdaten ausgibt,
und als mentale Messdaten die reaktiven Messdaten verwendet werden, wobei eine vom Patienten bedienbare Eingabevorrichtung, insbesondere in Form eines Touch-Screens (3), aufweist, wobei die reaktiven Messdaten zumindest teilweise den in die Eingabevorrichtung eingegebenen Eingabedaten entsprechen,
**dadurch gekennzeichnet, dass** die reaktiven Messdaten nicht nur aus der Eingabevorrichtung (3), sondern auch aus zumindest einer der folgenden Messvorrichtungen stammen:
- eine Elektrokardiographie-Einrichtung (EKG),
- eine Einrichtung zur Feinstrommessung,
- eine Einrichtung zur Messung der Herzratenvariabilität (HRV)
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Elektroenzephalographie-Einrichtung (EEG),
- eine Elektroneurographie-Einrichtung (ENG),
- eine Einrichtung zur Video-Erfassung des Ganges des Patienten,
und dass zumindest eine Therapievorrichtung (15) vorgesehen ist, der mindestens ein Teil der Ausgangssignale der elektronischen Auswerteinrichtung (5) als Steuersignale zugeführt werden, in deren Abhängigkeit die Therapievorrichtung ausgelegt ist, unterschiedlich zu arbeiten.

2. Einrichtung nach Anspruch 1, **dadurch gekennzeichnet, dass** die Messvorrichtungen zumindest zwei der folgenden Messvorrichtungen umfasst:
- eine Elektrokardiographie-Einrichtung (EKG),
- eine Einrichtung zur Feinstrommessung,
- eine Einrichtung zur Messung der Sauerstoffsättigung im Blut,
- eine Infrarot-Thermoscan-Einrichtung
- eine Einrichtung zum Blutdruckmessen,
- eine Einrichtung zur Messung der Herzratenvariabilität (HRV),
- eine Einrichtung zum Analysieren des Blutes,
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Einrichtung zur Untersuchung der Haut und/oder der Zähne mittels bildgebender Erfassung,
- eine Einrichtung zur Untersuchung und Analyse der Atemluft,
- eine Ultraschall-Messvorrichtung,
- eine Elektroenzephalographie-Einrichtung (EEG),
- eine Elektromyographie -Einrichtung (EMG),
- eine Elektroneurographie-Einrichtung (ENG),
- eine vom Patienten bedienbare Eingabevorrichtung,
- eine Einrichtung zur Video-Erfassung des Ganges des Patienten.

3. Einrichtung nach Anspruch 1 oder 2, **dadurch gekennzeichnet, dass** die körperlichen Messdaten aus zumindest einer der folgenden Messvorrichtungen stammen:
- eine Elektrokardiographie-Einrichtung (EKG),
- eine Einrichtung zur Feinstrommessung,
- eine Einrichtung zur Messung der Sauerstoffsättigung im Blut,
- eine Infrarot-Thermoscan-Einrichtung
- eine Einrichtung zum Blutdruckmessen,
- eine Einrichtung zur Messung der Herzratenvariabilität (HRV),
- eine Einrichtung zum Analysieren des Blutes,
- eine Einrichtung zur Frequenzanalyse von Organen,
- eine Einrichtung zur Messung von Photonen aus biologischem Gewebe,
- eine Einrichtung zur Magnet- und Impedanzmessung von biologischem Gewebe,
- eine Einrichtung zur Untersuchung der Haut und/oder der Zähne mittels bildgebender Erfassung,
- eine Einrichtung zur Untersuchung und Analyse der Atemluft,
- eine Ultraschall-Messvorrichtung,
- eine Elektromyographie-Einrichtung (EMG).

4. Einrichtung nach einem der Ansprüche 1 bis 3, **dadurch gekennzeichnet, dass** die Einrichtung zur Beeinflussung des Patienten einen Bildschirm (3) und/oder einen Lautsprecher umfasst.

5. Einrichtung nach einem der Ansprüche 1 bis 4, **dadurch gekennzeichnet, dass** zumindest eine Anzeigevorrichtung (14) für den Arzt, zumindest eine Anzeigevorrichtung (10) für den Patienten und/oder zumindest eine Druckvorrichtung (28) vorgesehen sind (ist), denen jeweils Ausgangssignale aus der elektronischen Auswerteinrichtung (5) zur graphisch aufbereiteten Anzeige und/oder Druck zugeführt werden.

6. Einrichtung zur Untersuchung eines menschlichen Patienten (1) mit einem Träger (2) für den Patienten (1), mindestens einer - insbesondere als Bildschirm ausgebildeten - Anzeigevorrichtung (3), die von dem auf dem Träger (2) befindlichen Patienten (1) einsehbar ist, mindestens einer Messvorrichtung (4) zum Erfassen von Messdaten des Patienten (1) sowie einer Auswerteinrichtung, die mit der Anzeigevorrichtung (3) und der Messvorrichtung (4) in Verbindung steht, nach einem der Ansprüche 1 bis 5, **dadurch gekennzeichnet, dass** zumindest eine Anzeigevorrichtung und zumindest ein Sensor (4a) einer Messvorrichtung (4) gegenüber dem Träger (2) für den Patienten (1) beweglich gelagert ist.

7. Einrichtung nach Anspruch 6, **dadurch gekennzeichnet, dass** zumindest eine Anzeigevorrichtung und zumindest ein Sensor (4a) einer Messvorrichtung (4) auf einem gemeinsamen Halter (6), insbesondere Schlitten, bewegbar gelagert sind.

8. Einrichtung nach einem der Ansprüche 6 oder 7, **dadurch gekennzeichnet, dass** die zumindest eine Messeinrichtung einen medizinischen Thermoscanner, eine - insbesondere zum Eye-Tracking geeignete -Kamera und/oder ein Mikrofon zum Aufnehmen von Lauten des Patienten umfasst.

9. Einrichtung nach einem der Ansprüche 6 bis 8, **dadurch gekennzeichnet, dass** die zumindest eine Anzeigevorrichtung (3) und der zumindest eine Sensor (4a) einer Messvorrichtung (4) motorisch bewegbar an einer Tragvorrichtung (9) gelagert ist.

10. Einrichtung nach einem der Ansprüche 6 bis 9, **gekennzeichnet durch** eine - insbesondere als Aufnahmefach ausgebildete - Aufnahme (12) für zumindest eine weitere Messvorrichtung, wobei die Aufnahme vorzugsweise einer Tragvorrichtung (9) ausgebildet ist, die auch die Anzeigevorrichtung und den Sensor an der Messvorrichtung beweglich trägt.

11. Einrichtung nach einem der Ansprüche 6 bis 10, **dadurch gekennzeichnet, dass** der Träger (2) für den Patienten (1) als mehrteilige Sitzliege ausgebildet ist, deren Teile in ihrer Lage - vorzugsweise motorisch - verstellbar sind, wobei die Sitzliege vorzugsweise dreiteilig mit einem Brustteil, einem Mittelteil und einem Fußteil ausgebildet ist.

12. Einrichtung nach Anspruch 11, **dadurch gekennzeichnet, dass** die Sitzliege in eine Sitzstellung und die Anzeigevorrichtung in eine Stellung bringbar sind, in der der Kopf des Patienten im Wesentlichen auf derselben Höhe liegt wie die Anzeigevorrichtung.

13. Einrichtung nach einem der Ansprüche 11 oder 12, **dadurch gekennzeichnet, dass** die Sitzliege und die Anzeigevorrichtung in eine Stellung bewegbar sind, in der der Patient im Wesentlichen geradeaus auf die Anzeigevorrichtung blickt.

## Claims

1. An apparatus for examining a human patient (1), having at least two, preferably multiple different measuring devices which provide measurement data on the patient's condition, wherein an electronic evaluation apparatus is provided that is adapted to determine output signals depending on measurement data from at least two different measuring devices and their interaction, wherein the electronic evaluation apparatus is supplied with, on the one hand, mental measurement data reflecting the patient's mental state, and, on the other hand, physical measurement data reflecting the patient's physical state, wherein the electronic evaluation apparatus (5) is adapted to determine the output signals from the mental measurement data and the physical measurement data and their interaction;
wherein the mental measurement data on the one hand and the physical measurement data on the other hand come at least partially from different measuring devices,
wherein an apparatus is provided for non-invasive and noncontact influencing of the patient and at least one measuring device is set up to record the patient's response and to output reactive measurement data dependent thereon,
and the reactive measurement data are used as mental measurement data, wherein said apparatus has an input device operable by the patient, in particular in the form of a touch screen (3), wherein the reactive measurement data correspond at least partially to the input data entered in the input device,
**characterized in that** the reactive measurement data do not only come from the input device (3), but also from at least one of the following measuring devices:
- an electrocardiography (ECG) apparatus,
- an apparatus for fine current measuring,
- an apparatus for measuring heart rate variability (HRV),
- an apparatus for organ frequency analysis,
- an apparatus for measuring photons from biological tissue,
- an apparatus for magnet and impedance measurement of biological tissue,
- an electroencephalography (EEG) apparatus,
- an electroneurography (ENG) apparatus,
- an apparatus for video recording the patient's gait, and **in that** at least one therapy apparatus (15) is provided to which at least part of the output signals of the electronic evaluation apparatus (5) is supplied as control signals, depending on which the therapy apparatus is adapted to operate differently.

2. The apparatus according to claim 1, **characterized in that** the measuring devices include at least two of the following measuring devices:
- an electrocardiography (ECG) apparatus,
- an apparatus for fine current measuring,
- an apparatus for measuring oxygen saturation in the blood,
- an infrared thermoscan apparatus,
- an apparatus for measuring blood pressure,
- an apparatus for measuring heart rate variability (HRV),
- an apparatus for analyzing blood,
- an apparatus for organ frequency analysis,
- an apparatus for measuring photons from biological tissue,
- an apparatus for magnet and impedance measurement of biological tissue,
- an apparatus for examining the skin and/or teeth by means of imaging,
- a apparatus for the examination and analysis of breathing air,
- an ultrasonic measuring device,
- an electroencephalography (EEG) apparatus,
- an electromyography (EMG) apparatus,
- an electroneurography (ENG) apparatus,
- a patient-operable input device,
- an apparatus for video recording the patient's gait.

3. The apparatus according to claim 1 or 2, **characterized in that** the physical measurement data come from at least one of the following measuring devices:
- an electrocardiography (ECG) apparatus,
- an apparatus for fine current measuring,
- an apparatus for measuring oxygen saturation in the blood,
- an infrared thermoscan apparatus,
- an apparatus for measuring blood pressure,
- an apparatus for measuring heart rate variability (HRV),
- an apparatus for analyzing blood,
- an apparatus for organ frequency analysis,
- an apparatus for measuring photons from biological tissue,
- an apparatus for magnet and impedance measurement of biological tissue,
- an apparatus for examining the skin and/or teeth by means of imaging,
- a apparatus for the examination and analysis of breathing air,
- an ultrasonic measuring device,
- an electromyography (EMG) apparatus.

4. The apparatus according to any one of claims 1 to 3, **characterized in that** the apparatus comprises a screen (3) and/or a speaker for influencing the patient.

5. The apparatus according to any one of claims 1 to 4, **characterized in that** at least one display device (14) for the physician, at least one display device (10) for the patient and/or at least one printing device (28) are provided, which are supplied with output signals from the electronic evaluation apparatus (5) for graphically processed display and/or printing.

6. An apparatus for examining a human patient (1) with a support (2) for the patient (1), at least one display device (3), particularly configured as a screen, which can be seen by the patient (1) on the support (2), at least one measuring device (4) for capturing the patient's measurement data (1), and an evaluation apparatus according to any one of claims 1 to 5 connected to the display device (3) and the measuring device (4), **characterized in that** at least one display device and at least one sensor (4a) of a measuring device (4) is movably mounted in relation to the support (2) for the patient (1).

7. The apparatus according to claim 6, **characterized in that** at least one display device and at least one sensor (4a) of a measuring device (4) are movably mounted on a common holder (6), particularly a carriage.

8. The apparatus according to any one of claims 6 or 7, **characterized in that** at least one measuring device comprises a medical thermoscanner, a camera, particularly one suitable for eye tracking, and/or a microphone for recording sounds of the patient.

9. The apparatus according to any one of claims 6 to 8, **characterized in that** the at least one display device (3) and the at least one sensor (4a) of a measuring device (4) motormovably mounted at a carrier device (9).

10. The apparatus according to any one of claims 6 to 9, **characterized by** a receptacle (12), particularly configured as a receiving compartment, for at least one further measuring device, wherein the receptacle is preferably configured as a carrier device (9) which also movably carries the display device and the sensor on the measuring device.

11. The apparatus according to any one of claims 6 to 10, **characterized in that** the support (2) for the patient (1) is configured as a multi-part seat bed, the positioning of the parts of which is adjustable, preferably motor-adjustable, wherein the seat bed is preferably configured in three parts with a chest part, a middle part, and a foot part.

12. The apparatus according to claim 11, **characterized in that** the seat bed can be moved to a seat position and the display device can be moved to a position in which the head of the patient is substantially at the same height as the display device.

13. The apparatus according to claims 11 or 12, **characterized in that** the seat bed and the display device can be moved to a position in which the patient is substantially looking straight ahead at the display device.

## Revendications

1. Dispositif servant à l'examen d'un patient humain (1), avec au moins deux, de préférence plusieurs dispositifs de mesure différents qui délivrent des données de mesure relatives à l'état du patient, dans lequel un dispositif d'évaluation électronique est prévu, lequel est conçu de sorte qu'il détermine en fonction de données de mesure provenant d'au moins deux dispositifs de mesure différents et leur corrélation des signaux de sortie, dans lequel au dispositif d'évaluation électronique sont fournies d'une part des données de mesure mentales qui reproduisent l'état psychique du patient, et d'autre part des données de mesure corporelles qui reproduisent l'état corporel du patient, dans lequel le dispositif d'évaluation (5) électronique est conçu de sorte qu'il détermine à partir des données de mesure mentales et des données de mesure corporelles et leur corrélation des signaux de sortie,
dans lequel les données de mesure mentales d'une part et les données de mesure corporelles d'autre part proviennent au moins partiellement de dispositifs de mesure différents, dans lequel un dispositif d'intervention non invasif et sans contact du patient est prévu et au moins un dispositif de mesure est orienté de sorte qu'il détecte la réaction du patient et délivre des données de mesure réactives qui en dépendent,
et les données de mesure réactives sont utilisées comme données de mesure mentales, dans lequel un dispositif de saisie utilisable par le patient, en particulier sous la forme d'un écran tactile (3), présente, dans lequel les données de mesure réactives correspondent au moins partiellement aux données de saisie saisies dans le dispositif de saisie,
**caractérisé en ce que** les données de mesure réactives proviennent non seulement du dispositif de saisie (3) mais aussi d'au moins un des dispositifs de mesure suivants :
- un dispositif d'électrocardiographie (ECG),
- un dispositif de mesure de courant fin,
- un dispositif de mesure de variabilité de fréquence cardiaque (VFC),
- un dispositif d'analyse de fréquence d'organes,
- un dispositif de mesure de photons en tissu biologique,
- un dispositif de mesure magnétique et d'impédance de tissu biologique,
- un dispositif d'électroencéphalographie (EEG),
- un dispositif d'électroneurographie (ENG),
- un dispositif de détection vidéo de la marche du patient, et **en ce qu'**au moins un dispositif thérapeutique (15) est prévu, l'au moins une partie des signaux de sortie est fournie au dispositif d'évaluation (5) électronique comme signaux de commande, en fonction desquels le dispositif thérapeutique est conçu afin de travailler différemment.

2. Dispositif selon la revendication 1, **caractérisé en ce que** les dispositifs de mesure comprennent au moins deux des dispositifs de mesure suivants :
- un dispositif d'électrocardiographie (ECG),
- un dispositif de mesure de courant fin,
- un dispositif de mesure de la saturation en oxygène dans le sang,
- un dispositif de Thermoscan à infrarouge,
- un dispositif de mesure de pression artérielle,
- un dispositif de mesure de la variabilité de fréquence cardiaque (VFC),
- un dispositif d'analyse du sang,
- un dispositif d'analyse de fréquence d'organes,
- un dispositif de mesure de photons de tissu biologique,
- un dispositif de mesure magnétique et d'impédance de tissu biologique,
- un dispositif d'examen de la peau et/ou des dents au moyen de la détection par imagerie,
- un dispositif d'examen et d'analyse de l'air de respiration,
- un dispositif de mesure à ultrasons,
- un dispositif d'électroencéphalographie (EEG),
- un dispositif d'électromyographie (EMG),
- un dispositif d'électroneurographie (ENG),
- un dispositif de saisie utilisable par le patient,
- un dispositif de détection vidéo de la marche du patient.

3. Dispositif selon la revendication 1 ou 2, **caractérisé en ce que** les données de mesure corporelles proviennent d'au moins un des dispositifs de mesure suivants :
- un dispositif d'électrocardiographie (ECG),
- un dispositif de mesure de courant fin,
- un dispositif de mesure de la saturation en oxygène dans le sang,
- un dispositif de Thermoscan à infrarouge,
- un dispositif de mesure de pression artérielle,
- un dispositif de mesure de la variabilité de fréquence cardiaque (VFC),
- un dispositif d'analyse du sang,
- un dispositif d'analyse de fréquence d'organes,
- un dispositif de mesure de photons de tissu biologique,
- un dispositif de mesure magnétique et d'impédance de tissu biologique,
- un dispositif d'examen de la peau et/ou des dents au moyen de la détection par imagerie,
- un dispositif d'examen et d'analyse de l'air de respiration,
- un dispositif de mesure à ultrasons,
- un dispositif d'électromyographie (EMG).

4. Dispositif selon l'une quelconque des revendications 1 à 3, **caractérisé en ce que** le dispositif de manipulation du patient comprend un écran (3) et/ou un haut-parleur.

5. Dispositif selon l'une quelconque des revendications 1 à 4, **caractérisé en ce qu'**au moins un dispositif d'affichage (14) pour le médecin, au moins un dispositif d'affichage (10) pour le patient et/ou au moins un dispositif d'impression (28) sont (est) prévus, auxquels respectivement des signaux de sortie du dispositif d'évaluation (5) électronique sont fournis pour l'affichage présenté graphiquement et/ou l'impression.

6. Dispositif servant à l'examen d'un patient humain (1) avec un support (2) pour le patient (1), au moins un dispositif d'affichage (3) - réalisé en particulier comme écran - qui peut être vu par le patient (1) se trouvant sur le support (2), au moins un dispositif de mesure (4) pour la détection de données de mesure du patient (1) ainsi qu'un dispositif d'évaluation qui est en liaison avec le dispositif d'affichage (3) et le dispositif de mesure (4), selon l'une quelconque des revendications 1 à 5, **caractérisé en ce qu'**au moins un dispositif d'affichage et au moins un capteur (4a) d'un dispositif de mesure (4) sont logés de manière mobile par rapport au support (2) pour le patient (1).

7. Dispositif selon la revendication 6, **caractérisé en ce qu'**au moins un dispositif d'affichage et au moins un capteur (4a) d'un dispositif de mesure (4) sont logés de manière mobile sur un support (6) commun, en particulier un chariot.

8. Dispositif selon l'une quelconque des revendications 6 ou 7, **caractérisé en ce que** l'au moins un dispositif de mesure comprend un thermoscanner médical, une caméra - appropriée en particulier à l'oculométrie et/ou un microphone pour la prise de sons du patient.

9. Dispositif selon l'une quelconque des revendications 6 à 8, **caractérisé en ce que** l'au moins un dispositif d'affichage (3) et l'au moins un capteur (4a) d'un dispositif de mesure (4) sont logés de manière mobile sous l'action d'un moteur au niveau d'un dispositif de support (9).

10. Dispositif selon l'une quelconque des revendications 6 à 9, **caractérisé par** un logement (12) - réalisé en particulier comme compartiment de réception - pour au moins un autre dispositif de mesure, dans lequel le logement de préférence d'un dispositif de support (9) est réalisé, lequel porte aussi de manière mobile le dispositif d'affichage et le capteur au niveau du dispositif de mesure.

11. Dispositif selon l'une quelconque des revendications 6 à 10, **caractérisé en ce que** le support (2) pour le patient (1) est réalisé comme lit pliable en plusieurs parties, dont les parties sont réglables dans leur position - de préférence de manière motorisée -, dans lequel le lit pliable est de préférence réalisé en trois parties avec une partie torse, une partie médiane et une partie pied.

12. Dispositif selon la revendication 11, **caractérisé en ce que** le lit pliable peut être amené dans une position d'assise et le dispositif d'affichage peut être amené dans une position, dans laquelle la tête du patient se trouve sensiblement à la même hauteur que le dispositif d'affichage.

13. Dispositif selon l'une quelconque des revendications 11 ou 12, **caractérisé en ce que** le lit pliable et le dispositif d'affichage sont mobiles dans une position, dans laquelle le patient regarde sensiblement tout droit sur le dispositif d'affichage.
